(19) Europäisches Patentamt
European Patent Office
Office européen des brevets

(11) EP 3 473 327 A1

(12) EUROPEAN PATENT APPLICATION

(43) Date of publication:
**24.04.2019 Bulletin 2019/17**

(21) Application number: **17197384.5**

(22) Date of filing: **19.10.2017**

(51) Int Cl.:
***B01D 61/28*** *(2006.01)*
***A61M 1/34*** *(2006.01)*
***A61M 1/36*** *(2006.01)*
***B01D 63/02*** *(2006.01)*
***B01D 69/02*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **3M Innovative Properties Company**
**Saint Paul, MN 55133-3427 (US)**

(72) Inventors:
- **Von Harten, Bodo**
**41453 Neuss (DE)**
- **Riesop, Peter**
**41453 Neuss (DE)**

(74) Representative: **Hettstedt, Stephan et al**
**3M Deutschland GmbH**
**3M Office of Intellectual Property Counsel**
**Carl-Schurz Str. 1**
**41453 Neuss (DE)**

# (54) INTEGRATED FLUID TREATMENT DEVICE, AND PROCESS USING SUCH DEVICE

(57) Device for multi-stage fluid treatment comprising first and second fluid treatment compartment being arranged in sequence. First compartment comprises a first cylindrical shell and a plurality of hollow fiber membranes arranged therein, which are embedded with their ends in a first and a second tube sheet bonded to the first shell at first and second ends of the first shell, such that a first outer space is formed around the hollow fiber membranes. The hollow fibers membranes pass with their ends through the tube sheets and are open at their ends. The first fluid treatment compartment has at least one opening in the first shell to permit fluid flow into or out of the first outer space. The second compartment comprises a second cylindrical shell and a plurality of fibers being arranged therein extending between a third end and a fourth end of the second shell. The fibers are at least embedded with one of their ends in a tube sheet bonded to the second shell at one of the ends of the second shell such that a second outer space is formed around the fibers which is in fluid communication with a fluid port in the second shell. The device is characterized in that first and second compartment are connected so as to form an integrated unit, wherein second end of second cylindrical shell and third end of second cylindrical shell of are fluid-tightly connected to each other such that at the transition between first compartment and second compartment a fluid path is formed between the lumina of the hollow fibers and the second outer space.

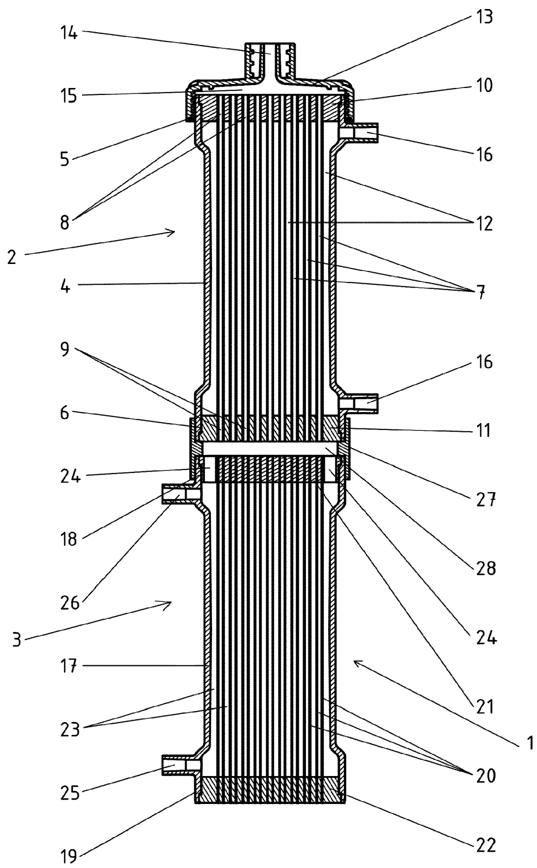

Fig. 1

EP 3 473 327 A1

## Description

The invention relates to a device for a multi-stage treatment of a fluid comprising at least a first blood treatment compartment and a second blood treatment compartment being arranged in sequence and in particular to a device for the treatment of sepsis.

Today a field of blood treatment gaining increasing importance relates to the purification of the blood of patients who exhibit a pathology that makes it necessary to reduce the circulating amounts of several substances having different physico-chemical characteristics. Crohn's disease, rheumatoid arthritis, certain forms of psoriasis or even Behcet's disease belong, i.a., to this category because the immune and inflammatory systems interact to create chronic inflammations that progress until greatly reducing the quality of life of patients. Sepsis and Systemic Inflammatory Response Syndromes (SIRS) are another group of diseases the treatment of which e.g. by membranes based treatment systems became more and more important during the last few years.

Sepsis and Systemic Inflammatory Response Syndromes (SIRS) are with mortality rates between 30-70% a leading cause of death in intensive care units. Sepsis is a disease that is characterized by a complex systemic inflammatory reaction of the organism to the penetration of infectious agents. The inflammatory response leads to organ dysfunction of varying degrees and often ends with the death of the patient. Affected patients die to a minor extend of the direct effects of bacterial infection, but above all of the systemic effects of the often excessive inflammatory response of the body. This immune response, in which the production of so-called pro-inflammatory mediators such as cytokines by neutrophils and monocytes plays a key role, is increasingly difficult to control with progression of sepsis.

A blood treatment system for the treatment of acute, by mediators mediated inflammatory diseases is disclosed in WO 2016/030357. This system is based on the approach that activated leukocytes contribute to the production of cytokines, which in turn activate other immune cells, which finally leads to systemic overreaction of the immune defence system and to sepsis. Thus, the system of WO2016/030357 shall allow combined removal of leukocytes as well as of toxic mediators like cytokines and comprises a first device and a second device with the first device being a membrane filter for the removal of toxic mediators from blood, and the second device being suitable for the removal of granulocytes and monocytes from blood. First and second device of WO 2016/03057 are connected in series such that the blood flows through first and second device successively.

While according to WO 2016/030357 the first device in each case is a membrane filter with a separation characteristic such that the sieving coefficient for albumin in the range from 0,015 to 0,35, the second device in preferred embodiments may be a filter or an adsorber, e.g. based on a fibrous or particulate material.

US 2009/0060890 and US2012/0322138 disclose an integrated two stage module in a single treatment device for selective cytopheresis, in which cells like leukocytes and platelets are removed from blood. In this two-stage module blood enters one end of the first stage of the module and travels through first hollow fibers arranged in the first stage, whereby ultrafiltrate passes through the walls of the hollow fibers into a hollow space surrounding the hollow fibers in the first stage. The filtered blood from the hollow fibers in the first stage passes into a hollow space in the second stage of the module and surrounds second hollow fibers arranged in the second stage which second hollow fibers are open at both their ends and contain ultrafiltrate, which is passed from the hollow space of the first stage through the lumina of the second hollow fibers. The blood flows over the hollow fibers arranged in the hollow space of the second stage and filled with ultrafiltrate, and leukocytes are sequestered on the second hollow fibers while blood and ultrafiltrate can interact with one another across the walls of the hollow fiber membranes. Blood ultimately exits the module at a side port, which is in fluid connection with the hollow space of the second stage, and ultrafiltrate may exit as waste via an end port or may at least partially be returned to the blood flowing over the hollow fibers of the second stage via the walls of these hollow fibers However, US 2009/0060890 and US 2012/0322138 do not address removal of different species from a fluid like e.g. toxic mediators and leukocytes from a blood sample. Moreover, the modules disclosed in these documents are not suitable e.g. for the combined removal of removal of toxic mediators and of leukocytes from blood.

Thus, there is a need for an integrated multi-compartment device for the treatment of fluids that allows different fluid treatments in sequence. The integrated device shall allow simply handling and shall allow a variety of different fluid treatments. In particular, there is a need for an integrated device for combined removal of different species from blood and more particular for an integrated device for combined removal of toxic mediators and leukocytes from blood for the treatment of sepsis. The problem to be solved by the present invention is therefore to provide such a device.

The problem is solved by a device for multi-stage treatment of a fluid comprising at least a first fluid treatment compartment and a second fluid treatment compartment being arranged in sequence,

- the first fluid treatment compartment comprising

  - a first cylindrical shell having a first end and a second end,
  - a plurality of first hollow fiber membranes based on an organic polymer, each said first hollow fiber membranes having a first end and a second end, a lumen and a wall surrounding the lumen,

- wherein said first hollow fiber membranes are arranged in the first cylindrical shell and are extending between the first end and the second end,
- wherein said first hollow fiber membranes are embedded with their first end in a first tube sheet bonded to an interior side of the shell at the first end of the first shell and with their second ends in a second tube sheet bonded to the interior side of the first shell at the second end of the first shell, such that a first outer space is formed around the first hollow fiber membranes which is limited by the interior side of the first shell and the first and second tube sheets,
- wherein the first hollow fiber membranes pass with their ends through the respective tube sheets and are open at their ends,
- wherein the first fluid treatment compartment has attached to the first end of the first shell a first end cap with a first fluid port, which first fluid port being in fluid connection with the lumina of the first hollow fiber membranes at their first ends via a first headspace formed between first end cap and first tube sheet,
- wherein the first fluid treatment compartment has at least one opening in the first shell between first and second tube sheets to permit fluid flow into or out of the first outer space surrounding the first hollow fiber membranes,

- the second fluid treatment compartment comprising

  - a second cylindrical shell having a third end and a forth end, wherein the fourth end of the second cylindrical shell being delimited by an end piece,
  - a plurality of fibers based on an organic polymer which fibers having a length extension and ends,
  - wherein said fibers are arranged in the second cylindrical shell such that they extend between third and fourth end of the second cylindrical shell and
  - wherein the fibers are at least embedded with one of their ends in a tube sheet bonded to an interior side of the second shell at one of the ends of the second shell such that a second outer space is formed around the fibers which is limited at least by the second shell and the end piece at the fourth end of the second shell,
  - wherein the second fluid treatment compartment has at least one fluid port, which at least one fluid port of the second fluid treatment compartment being in fluid communication with the second outer space and being adapted to permit fluid flow into or out of the second outer space surrounding the fibers arranged in the second shell,

wherein the device is characterized in that first fluid treatment compartment and second fluid treatment compartment are connected so as to form an integrated unit, wherein second end of first cylindrical shell of and third end of second cylindrical shell are fluid-tightly connected to each other such that at the transition between the first fluid treatment compartment and the second fluid treatment compartment a fluid path is formed between the lumina of the hollow fibers of the first fluid treatment compartment and the second outer space of the second fluid treatment compartment.

With the device of the invention, a cascading treatment, i.e. a treatment in consecutive manner of a fluid flowing through the device can be performed , e.g. with a first fluid treatment step in form of a filtration and a second step in form of an adsorptive treatment or vice versa depending from the flow direction through the device. The fluid may first pass through the device of the invention in such a way that it first flows through first fluid port in the first end cap into the first headspace and from there into the lumina of the hollow fibers of the first fluid treatment compartment. Part of the fluid flowing through the lumina of the first hollow fibers may flow as permeate through the walls of the first hollow fibers into the first outer space of the first fluid treatment compartment. The permeate may leave the first outer space via a side port in the first cylindrical shell positioned between first and second tube sheet.

The retentate flows from the lumina of the first hollow fibers into the second outer space of the second fluid treatment compartment and flows over the plurality of fibers in the second cylindrical shell of the second fluid treatment compartment. When flowing over the fibers in the second fluid treatment compartment, the retentate or fluid to be treated may be treated in a second step e.g. by an adsorption process. The adsorption may comprise, for example, an interaction with functional groups, which may be attached to the fibers. After the second treatment, the twice-treated fluid leaves the second outer space of the second fluid treatment compartment via the at least one fluid port of the second fluid treatment compartment.

Of course, when appropriate, a fluid to be treated can flow through the device of the invention also in reverse order, i.e. it can flow at first through the second fluid treatment compartment and then through the first fluid treatment compartment. In this case, the fluid to be treated flows into the device of the invention via the fluid port of the second treatment compartment into the second outer space and flows over the fibers arranged in the second fluid treatment compartment. During contact with these fibers, the fluid to be treated may undergo a treatment process e.g. in form of an adsorption process.

From the second outer space in the second treatment compartment, the fluid flows into the lumina of the first hollow fibers of the first fluid treatment compartment, flows through the lumina and then into the first headspace formed between first end cap and first tube sheet of the first treatment compartment. During flow through the lumina of the first

hollow fiber membranes part of the fluid may be filtered as permeate through the walls of the hollow fiber membranes into the first outer space. The permeate may leave the first treatment compartment via a side port in the first cylindrical shell, while the retentate flows out of the lumina into the first headspace and leaves the device via the first fluid port.

As becomes evident from these explanations, at the connection of first and second treatment compartment a change in the direction of flow is effected in that the fluid to be treated is guided from a flow though lumina of the first hollow fibers in the first treatment compartment into the outer space around the fibers in the second treatment compartment or vice versa. Thus, at the transition between first and second fluid treatment compartment a change from luminar to extraluminar flow or vice versa is effectuated.

The first fluid treatment compartment comprises a plurality of first semipermeable hollow fiber membranes. Preferably, the wall thickness of the hollow fiber membranes may be in the range from 5 to 200 μm. The necessary mechanical stabilities can generally no longer be assured below a wall thickness of 5 μm, while with wall thicknesses above 200 μm the achievable transmembrane flows decrease significantly and the membrane surface in the first compartment becomes too low. It is an advantage if the first hollow fiber membranes according to the invention have a wall thickness in the range from 20 to 150 μm, and particularly advantageous if the wall thickness lies in the range from 30 to 100 μm. In order to achieve a good flow through the lumina of the hollow-fibre membranes in the application, particularly a favourable pressure drop, the inside diameter of the hollow fibre membranes according to the invention preferably may be in the range from 100 to 500 μm, and especially preferably in the range from 150 to 400 μm.

In order to ensure an adequate permeability, the porosity of the hollow fiber membranes according to the invention preferably may be above 60 vol.%. On the other hand, excessively high volume porosities are a disadvantage due to the loss of mechanical stability, so that the porosity generally lies preferably below 90 vol.%. The hollow fiber membranes according to the invention especially preferably have a volume porosity in the range of 70 to 85 vol.%.

The semipermeable first hollow fiber membranes in the first fluid treatment compartment are preferably hydrophilic membranes. In this context, an especially preferred embodiment of a hydrophilic membrane is made of a hydrophobic first polymer that is combined with a hydrophilic second polymer. Possibilities for the first polymer include technical plastics from the group of aromatic sulfonated polymers, such as polysulfone, polyethersulfone, polyphenylenesulfone or polyarylethersulfone, the polycarbonates, polyimides, polyetherimides, polyetherketones, polyphenylene sulfides, copolymers or modifications of these polymers or mixtures of these polymers. In an especially preferred embodiment, the hydrophobic first polymer is a polysulfone or a polyethersulfone having the repeating molecular units illustrated in formulas (I) and (II) below

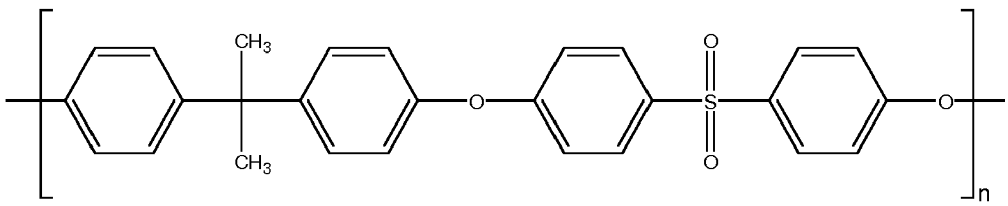

(I)

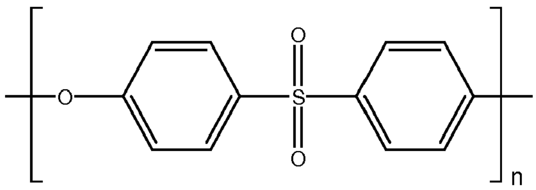

(II)

Long-chain polymers that, on the one hand, have a compatibility with the synthetic first polymer and that have repeating polymer units, which are themselves hydrophilic, are advantageously used as a hydrophilic second polymer. Preferably, the hydrophilic second polymer is polyvinylpyrrolidone, polyethylene glycol, polyvinyl alcohol, and polyglycolmonoester, polysorbitate, such as polyoxyethylene sorbitan monooleate, carboxylmethyl cellulose, or a modification or copolymer of these polymers. Polyvinylpyrrolidone is especially preferable.

The fibers in the second fluid treatment compartment may be hollow fibers, semipermeable hollow fiber membranes (in the following collectively named hollow fibers) or solid fibers. In a preferred embodiment, the fibers may be solid fibers. Solid fibers offer the advantage that flow occurs along the external surface only, i.e. there is no penetration into the wall like it may happen e.g. with hollow fiber membranes.

The fibers of the second compartment are arranged in the second cylindrical shell such that they extend between third and fourth end of the second cylindrical shell and that they have a high degree of order. The fibers lie in a similar arrangement with each other, i.e. a large proportion of the fibers are arranged next to each other along their extension

direction. The fibers are not arranged like non-woven fabrics, random fibers, or random laid fiber mats. Instead the arrangement of the fibers has a regular structure. In one preferred embodiment, the fibers are arranged in a bundle of straight fibers, which lie parallel to each other, or of corrugated or of crimped fibers, wherein the fibers all show the same extension direction. Likewise an arrangement is preferred in which at least 30% of the fibers lie parallel. This includes fibers that are present in several layers, whereby the fibers are arranged essentially parallel to each other within each layer. The parallel fibers in one layer can, however, may cross the parallel fibers of another layer. Arrangements of this type are described in EP-A-0 285 812. In comparison e.g. with non-woven fabrics, the arrangement of the fibers in accordance with the invention, wherein the fibers extend between third and fourth end of the second cylindrical shell, shows a greater surface area and a more controlled flow around the fibers of a fluid to be treated. Additionally, the arrangement of the fibers according to the invention with a high degree of order ensures that the formation of dead spaces and preferred channels, so called shunts, is largely prevented.

In one embodiment of the invention, the fibers in the second cylindrical shell may be arranged in U-shape. In this case, they may have terminal ends and a closed U-shaped fiber end opposite the terminal ends. It may also possible that the fibers are woven into a fabric as weft yarns with U-shaped bends at the selvedge. For U-shaped fibers, the terminal ends of the U-shaped fibers or the closed U-shaped fiber ends may be embedded in the tube sheet bonded to the interior side of the second shell.

The number of fibers in the second cylindrical shell preferably may be in the range from 2000 to 20,000 fibers, particularly preferably in the range from 4000 to 14,000 fibers). The outer diameter of the fibers preferably should be in the range from 0.05 mm to 2 mm, preferably from 0.075 mm to 2 mm, and particularly preferably from 0.1 mm to 1 mm.

Fibers made from natural polymers or from polymers that were produced synthetically are considered fibers made from organic polymers. Fibers made from natural polymers are particularly those based on cellulosic polymers, which also comprises fibers that are subject to the so-called polymer-analog reaction. Examples of such fibers based on cellulose are those made from regenerated cellulose, cellulose acetate, or modified cellulose, such as, e.g., cellulose ester, cellulose ether, cellulose modified with benzyl groups (benzyl cellulose) or cellulose modified with diethylaminoethyl or mixtures of these cellulosic polymers. In the method of the invention, a large reduction in the number of leukocytes is achieved with fibers based on cellulosic polymers; a particularly large reduction is obtained with fibers made of regenerated cellulose. Furthermore, fibers based on chitin or chitosan can be used.

Organic polymers also understood to be polymers that are produced using synthetic means. Fibers made of synthetic polymers of the following type can be used: those that consist of polyolefins, polyamides, polyacrylonitrile, polycarbonates or polyesters as well as modifications, blends, mixtures or copolymers of these polymers. Preferably, those polymers are used that are based on sulfone polymers, such as polysulfone or polyether sulfone. These polymers can be admixed with additional polymers like polyethylene oxide, polyhydroxyether, polyethylene glycol, polyvinyl alcohol or polycaprolactone as additives. The fibers can have, in addition to this, a coating with an additive. The fibers preferably contain as well a hydrophilizing agent, e.g., polyvinylpyrrolidone, or a hydrophilic modification of this polymer.

In one preferred embodiment, the fibers in the second fluid treatment compartment may be embedded with their ends facing the third end of the second cylindrical shell in a third tube sheet bonded to an interior side of the second shell at the third end of the second shell such that the second outer space around the fibers is limited by the second shell, the third tube sheet and the end piece at the fourth end of the second shell. The third tube sheet has an interior face facing the second outer space and an exterior face facing in direction of the second end of the first cylindrical shell. The second end of first cylindrical shell and third end of second cylindrical shell are fluid-tightly connected to each other such that a second headspace is formed between second tube sheet and third tube sheet. The third tube sheet has at least one opening therethrough forming a fluid path between the second headspace and the second outer space so that the lumina of the first hollow fiber membranes of the first fluid treatment compartment, the second head space and the second outer space are in fluid communication. Hereby, a fluid to be treated, which e.g. has entered the device of the invention via the first fluid port in the first end cap and has run through the lumina of the first hollow fiber membranes, flows out of the lumina of the first hollow fibers into the second headspace between second and third tube sheet. From there, it streams through the at least one opening in the third tube sheet into the second outer space surrounding the fibers in the second treatment compartment. The fluid - after having been treated in the second treatment compartment - is finally removed from the second treatment compartment via the fluid port of the second treatment compartment.

The at least one opening in the third tube sheet may be in form of one or more through-holes or bores in the third tube sheet. The at least one opening may have any contour. It may e.g. have a circular, an oval, a slit-like or a rectangular contour or it may be at least one segment of an annular slot. Preferably, the device comprises a multitude of openings in the third tube sheet distributed between the fibers embedded in the third tube sheet. In a preferred embodiment, the at least one opening is formed by at least one tube being embedded together with the fibers in the third tube sheet, which at least one tube extending through the third tube sheet and being open at both faces of the third tube sheet. In a preferred embodiment of the invention the at least one through-hole, bore or tube in the third tube sheet may have an inner hydraulic diameter $d_h$ in the range from 1 to 20 mm. In a more preferred embodiment the hydraulic diameter $d_h$ of the at least one opening is at most equal to the sum of the hydraulic diameters of the lumina of the first

hollow fiber membranes in the first fluid treatment compartment. The hydraulic diameter is defined as $d_h = 4 \cdot A/U$, wherein A is the area of the flow cross section of the at least one opening and the hollow fiber membrane lumen, respectively, and U is the circumference of the flow cross section of the at least one opening and the hollow fiber membrane lumen, respectively.

In a further preferred embodiment, the third tube sheet comprises a central sealing section embedding, in which the ends of the fibers of the second fluid treatment compartment are embedded and affixed, and a ring section surrounding the central sealing section, wherein a plurality of openings is arranged in the ring section. In a likewise preferred embodiment, a central perforated core tube having a hollow core, an open end and a closed end may form the at least one opening. This central perforated core tube may be embedded into the third tube sheet with its open end being in fluid communication with the second headspace, whereby the core of the central perforated core tube is adapted to communicate with the second outer space via its perforations. In this case, a fluid to be treated e.g. after having left the first fluid treatment compartment flows into the second headspace and from there via its open end into the core of the central perforated tube. Thereafter, the fluid to be treated leaves the central perforated tube via the perforations in the tube wall, enters the second outer space and flows across or along the fibers arranged in the second fluid treatment compartment. In cases of the fluid port of the second treatment compartment being a side port attached to the second shell between third tube sheet and closed fourth end the fluid flows through the second outer space in a radial flow.

In case of the fibers in the second treatment compartment being hollow fibers, the ends of the hollow fibers facing the third end of the second cylindrical shell preferably are embedded into the third tube sheet such that these ends are closed at the third tube sheet, i.e. that the lumina of the hollow fibers are not accessible for a fluid. This can be achieved e.g. by letting the ends terminate within the third tube sheet.

In a preferred embodiment of the device of the invention having the fibers in the second treatment compartment embedded with their ends facing the third end of the second cylindrical shell in a third tube sheet, the fibers may in addition be embedded with their ends facing the fourth end of the second cylindrical shell in a fourth tube sheet bonded to an interior side of the second shell. The fourth tube sheet forms the end piece at the fourth end of the second shell. In this case, the second outer space is limited by the second shell and by the third and the fourth tube sheets. In case the fibers in the second fluid treatment compartment being hollow fibers, it is preferred for the ends of the hollow fibers embedded in the fourth tube sheet to be closed at the fourth tube sheet, too, e.g. by letting the fiber ends terminate within the fourth tube sheet. From this, it follows that the lumina of the hollow fibers are also not accessible for the fluid to be treated at this end.

In case of U-shaped fibers, the opposite ends may be embedded in the third and the fourth tube sheet, respectively, regardless of whether the ends are terminal ends or closed ends. Again, in case of the fibers being hollow fibers it is preferred that terminal ends are closed e.g. by embedding these ends within the tube sheets.

For those embodiments of the device of the invention, for which the fibers are embedded also in a fourth tube sheet at the fourth end of the second cylindrical shell, the fourth tube sheet may close the fourth end of the second shell fluid-tightly. In this case, the at least one fluid port of the second fluid treatment compartment may be a side port attached to the second cylindrical shell between third tube sheet and fourth tube sheet.

In a further embodiment with the fibers in the second treatment compartment being embedded with their ends in tube sheets at the third and at the fourth end of the second cylindrical shell, the fourth tube sheet may comprise at least one opening or tube extending therethrough. As to possible embodiments of the at least one opening in the fourth tube sheet in principal the same embodiments can be considered as for the at least one opening in the third tube sheet. For these embodiments of the fourth tube sheet, the second cylindrical shell may be closed at the fourth end by a second end cap attached to the fourth end with a third headspace being formed between second end cap and fourth tube sheet and with the fluid port of the second treatment compartment being attached to the second end cap. Via the third headspace and the at least one opening in the fourth tube sheet this fluid port is in fluid communication with the second outer space and allows a fluid to flow into or out of the second outer space surrounding the fibers arranged in the second shell. In this case, a fluid streaming through the second outer space may penetrate from the second outer space through at least one opening in the fourth tube sheet into the third headspace formed between fourth tube sheet and second end cap and leave the second treatment compartment via the fluid port in the second end cap. Of course, also flow of the fluid in the opposite direction is possible, i.e. flow via the fluid port into the third headspace and through the openings or tubes in the fourth tube sheet into the second outer space.

In another preferred embodiment of the device having the fibers in the second treatment compartment embedded with their ends facing the third end of the second cylindrical shell in a third tube sheet the fiber ends facing the fourth end of the second cylindrical shell may terminate within the second outer space and may be free of an embedding in this case. Thus, the fiber ends facing the fourth end of the second cylindrical shell are not embedded e.g. in a tube sheet. In case of hollow fibers, the free ends of the hollow fibers terminating in the second outer space preferably are closed, e.g. by glueing, by hot melt sealing or by welding, so that a fluid present in the second outer space may not enter the lumina of the hollow fibers. In particular in case the fibers terminate within the second outer space, it is preferred to have a high filling ratio of the fibers in the second cylindrical shell in order to have a stable positioning of the fibers during flow

of a fluid to be treated through the second outer space. In addition, it is of advantage to keep the fibers apart from each other by known measures like e.g. by appropriate spacers or an appropriate make-up of the fibers in form of a bundle.

In case of the fiber ends directed to the fourth end of the second shell and terminating in the second outer space, the fourth end of the second cylindrical shell may be closed by a fourth tube sheet, which, however, does not embed the fibers, this fourth tube sheet forming the end piece. The fourth end of the second cylindrical shell may also be closed fluid-tightly by a second end cap attached to the fourth end of the second cylindrical shell, which second end cap then forms the end piece at the fourth end of the second cylindrical shell.

In embodiments for which the fibers ends directed to the fourth end of the second shell terminate within the second outer space, the at least one fluid port of the second compartment may be a side port attached to the second cylindrical shell between third tube sheet and fourth end of the second cylindrical shell or may be attached to the second end cap in case of a second end cap being present.

In embodiments, in which the fibers in the second cylindrical shell are arranged in U-shape, the U-shaped fibers may be embedded with their terminal ends in the third tube sheet while the closed U-shaped fiber ends directed towards the fourth end of the second cylindrical shell, terminate in the second outer space. The U-shaped fibers may, however, also be embedded with their closed U-shaped fiber ends in the third tube sheet, while their opposite ends are directed towards the fourth end of the second cylindrical shell and terminate in the second outer space. In case of U-shaped hollow fiber, terminal ends of the hollow fiber present in the second outer space preferably are closed so that the lumina of the hollow fibers are not accessible for a fluid flowing through the second outer space.

In a further preferred embodiment of the device of the invention, the tube sheet into which the fibers are embedded at one of the ends of the second cylindrical shell may be a fourth tube sheet bonded to an interior side of the second shell at the fourth end of the second cylindrical shell. Into this fourth tube sheet, the fibers are embedded with their ends facing the fourth end of the second shell, whereas the ends of the fibers facing the third end of the second cylindrical shell terminate within the second outer space and are free of an embedding. The fourth tube sheet forms the end piece at the fourth end of the second cylindrical shell.

In this embodiment, second end of first cylindrical shell and third end of second cylindrical shell are fluid-tightly connected to each other such that the second cylindrical shell, the fourth tube sheet and the second tube sheet limit the second outer space formed around the fibers. Moreover, the lumina of the first hollow fiber membranes of the first treatment compartment are in direct fluid communication with the second outer space and the first hollow fiber open directly into the second outer space of the second treatment compartment.

Preferably, the fourth tube sheet may close the fourth end of the second cylindrical shell fluid-tightly and the at least one fluid port of the second fluid treatment compartment may be a side port attached to the second cylindrical shell between third end of second cylindrical shell and fourth tube sheet. It is likewise preferred that the fourth tube sheet may comprise at least one opening therethrough and a second end cap being attached to the fourth end of the second cylindrical shell such that a third headspace is formed between fourth tube sheet an second end cap. In this case, second outer space and third headspace are in fluid communication via the at least one opening in the fourth tube sheet and the at least one fluid port of the second fluid treatment compartment is attached to the second end cap.

As to possible embodiments of the at least one opening in the fourth tube sheet in principal the same embodiments can be considered as for the at least one opening in the third tube sheet. Via the at least one opening in the fourth tube sheet the fluid port of the second fluid treatment compartment is in fluid communication with the second outer space and allows a fluid flow into or out of the second outer space. A fluid streaming through the second outer space may penetrate from the second outer space through at least one opening in the fourth tube sheet into the third headspace and leave the second treatment compartment via the fluid port in the second end cap. Of course, also flow of the fluid in the opposite direction is possible, i.e. flow via the fluid port into the third headspace and through the openings or tubes in the fourth tube sheet into the second outer space.

As already described with respect to embodiments of the device according to the invention with the fibers of the second fluid treatment compartment being embedded in a third tube sheet only and the fiber ends directed to the fourth end terminating within the second outer space, also for the present embodiments with embedding of the fibers in a fourth tube sheet only it is preferred to have a high filling ratio of the fibers in the second cylindrical shell. This ensures stable positioning of the fibers during flow of the fluid to be treated through the second outer space. In addition, it is also of advantage to keep the fibers apart from each other by known measures like e.g. by appropriate spacers or an appropriate make-up of the fibers in form of a bundle.

In case of hollow fibers, the free ends of the hollow fibers terminating in the second outer space preferably are closed, e.g. by glueing, by hot melt sealing or by welding, so that no fluid being present in the second outer compartment may enter the hollow fibers via their lumina.

Also in embodiments, in which the fibers of the second fluid treatment compartment are embedded in a fourth tube sheet only, positioned at the fourth end of the second cylindrical shell, fibers may be employed which are arranged in U-shape. The U-shaped fibers may be embedded with their terminal ends in the fourth tube sheet and the closed U-shaped fiber ends directed towards the third end of the second cylindrical shell may terminate in the second outer space.

The U-shaped fibers may, however, also be embedded with their closed U-shaped fiber ends in the fourth tube sheet, while their opposite ends directed towards the third end of the second cylindrical shell may terminate in the second outer space. In case of U-shaped hollow fibers, terminal ends of the hollow fibers present in the second outer space preferably are closed so that the lumina of the hollow fibers are not accessible for a fluid flowing through the second outer space.

For the first cylindrical shell and/or second cylindrical shell various cross sections are possible when looked along their length axis. The cross section may be round, oval, quadratic, rectangular etc. Preferably the cross section may be round as is known e.g. from dialyzers, plasmaseparators etc..

First and second fluid treatment compartments are connected to form an integrated unit. i.e. first and second fluid treatment compartment are joined together to form a single unit with two compartments, in which first fluid treatment compartment and second treatment compartment are directly attached to one another. In this respect, first and second cylindrical shell may be permanently joined together to form the single unit via an adhesive or a welded joint, for example. They may also be joint in a detachable manner by a screwed joint, e.g. in form of a coupling ring, or by a flanged joint or a Victaulic coupling.

The device of the invention is suitable for various multi-stage treatments of fluids. Depending on the particular application, the characteristics of the first hollow fiber membranes in the first compartment and of the fibers in the second compartment have to be adapted. One preferred embodiment of the device of the invention is a device for a multi-stage treatment of blood for treatment of sepsis aiming at removal of proinflammatory mediators and of granulocytes and monocytes. In this device, proinflammatory mediators such as cytokines are removed in the first fluid treatment compartment by a filtration process, and granulocytes and monocytes are removed in the second fluid treatment compartment by an adsorption process.

Thus, preferably the hollow fiber membranes of the first fluid treatment compartment are adapted to remove cytokines by filtration from a blood sample flowing through the lumina of the hollow fiber membranes. Likewise preferably, the fibers of the second fluid treatment compartment are adapted to remove granulocytes from blood by adsorption from a blood sample flowing through the second outer space.

In this respect, the first hollow fiber membranes preferably have a sieving coefficient for albumin in blood $SK_{Alb}$ within the range from 0.015 to 0.35, more preferably within the range from 0.05 to 0.3, and especially preferably within the range from 0.1 to 0.25. Thus, the semipermeable first hollow fiber membranes let through certain proportions of albumin to which toxic mediators or cytokines may be bonded at that time. Hollow fiber membranes having sieving coefficients of this type exhibit a separation limit in the range from 50,000 to 150,000 Daltons.

In another preferred embodiment, immunoglobulin G (IgG) having a molecular weight of approx. 180,000 Daltons is nearly completely retained by the semipermeable first hollow fiber membranes. The membranes thus preferably have a sieving coefficient for IgG $SK_{IgG}$ within the range from 0.001 to 0.1 and especially preferably within the range from 0.003 to 0.08.

The first hollow fiber membranes thus differ from e.g. plasma filters which are frequently used in the field of blood purification, which have a separation limit exceeding approximately two million Daltons, and in which a nearly complete separation of the aforementioned components dissolved in the blood plasma from the blood cells occurs via a separation of blood plasma. The plasma filtration membranes contained in plasma filters have a much more open structure than the first hollow fiber membranes of the device of the invention for the above-mentioned preferred application. This more open structure of the plasma filtration membranes simultaneously results in high permeabilities , resulting in ultrafiltration rates for water, $UFR_{water}$, in excess of approx. 15,000 ml/(h $m^2$ mmHg).

On the other hand, the membrane used in the first compartment for this preferred application differs from membranes used in hemodialyzers, hemodiafilters or hemofilters. The separation limit membranes for those applications - at up to 40,000 Daltons in whole blood - is designed in such a way that they at least nearly completely retain albumin and molecules larger than albumin, and for which sieving coefficients for albumin in blood $SK_{Alb}$ of less than 0.005 are realized.

Preferably, the first hollow fiber membranes for the preferred application respectively for the preferred device have an ultrafiltration rate in water or hydraulic permeability $UFR_{water}$ within the range from 500 to 2000 ml/(h $m^2$ mmHg). Especially preferably, the hydraulic permeability is within the range from 500 to 1500 ml/(h $m^2$ mmHg). Most suitable is a first membrane having an $UFR_{water}$ with the range from 800 to 1200 ml/(h $m^2$ mmHg). In this way, a sufficiently fast removal of toxic mediators during the blood treatment is achieved.

The determination of parameters for the characterization of the hollow fiber membranes of is based on the following measurement methods:

<u>Determination of the sieving coefficient for the hollw fiber membranes:</u>

The sieving coefficients SK are determined for $\alpha_1$ acidic glycoprotein ($M_w$= 44,000 Daltons), $SK_{Gp}$, albumin (Mw=68,000 Daltons), $SK_{Alb}$, and for immunoglobulin G ($M_W$=180,000 Daltons), $SK_{IgG}$. The determination of the sieving coefficients is carried out according to DIN EN ISO 8637:2014-03, in particular section 5.6.2 and figure 5, with freshly

donated human heparin blood (10 IU/ml) on a dialysis machine (Nikkiso DBB-03), wherein the whole blood is recirculated during the measurement. The blood is set to a hematocrit of 32% and a total protein concentration of 60 g/L prior to the experiment. The determination of the hematocrit takes place using a cell counting device (e.g. ABC Pentra 60, Axon Lab Ag), and the determination of the total protein concentration takes place using a clinical analyzer (e.g. Cobas c 111, Roche Diagnostics).

The membrane filter is first rinsed with 1 liter of saline in a single pass and then with another liter of saline on a recirculating basis (20 min, 200 ml/min). In the second rinsing step, rinsing liquid is extracted via the membrane filter into the filtrate chamber (60 ml/min) by means of an external pump (MPC, Ismatec). The saline is completely displaced by the blood, and the experiment is started at 37°C with blood flow $Q_B$=300 ml/min and a filtrate flow $Q_F$=60 ml/min (= 20% of the blood flow). After 60 minutes, the specimens of blood input and output as well as a filtrate specimen are drawn, plasma is extracted from them by centrifugation, and the concentrations of $\alpha_1$ acidic glycoprotein, albumin and IgG are determined via laser nephelometry (BN ProSpec, Siemens Diagnostics). The sieving coefficient occurs as described in section 5.6.2.4 of DIN EN ISO 8637:2014-03.

Separation limit:

To determine the separation limit, the sieving coefficients determined according to the previously described method for $\alpha_1$ acidic glycoprotein ($M_w$= 44,000 Daltons), $SK_{Gp}$, albumin ($M_w$=68,000 Daltons), $SK_{Alb}$, and for immunoglobulin G (Mw=180,000 Daltons), $SK_{IgG}$, are plotted in a diagram over the molecular weight. With integration of assumed vertices at 10,000 Daltons with a sieving coefficient SK=1 and at 1 million Daltons with a sieving coefficient SK=0, a compensation curve goes through the points. As a separation limit, the molecular weight is determined at a retention of 95% or a sieving coefficient SK of 0.05.

Ultrafiltration rate in water $UFR_{water}$ (hydraulic permeability):

To determine the hydraulic permeability or the ultrafiltration rate in water $UFR_{water}$ of the semipermeable hollow fiber membrane, a test cell having a defined number of hollow fibers and length is prepared from the hollow fiber membranes to be tested. For this purpose, the hollow fibers are embedded in hot wax on both sides at their ends. After the wax hardens, the embeddings are cut out so that the lumina of the hollow fiber membranes are opened by the cut. The hollow fiber lumina in the embeddings must be checked for continuity. The length of the test cell is typically 300 +/- 5 mm. The number of hollow fiber membranes is generally between 160 - 240.

The effective surface of a test cell is defined as follows:

$$A = n \cdot l \cdot d_i \cdot \pi \cdot f_{dim} \quad [m^2]$$

where

A = effective area [$m^2$]
n = number of capillaries
l = free length of the capillaries [mm]
di = inner diameter of the capillaries [$\mu$m]
$f_{dim}$ = dimension factor [$1 \cdot 10^{-9}$ $m^2$ /(mm$\cdot\mu$m)]

Before the measurement, the test cell is stored at room temperature in deionized water (wetting) for at least 15 minutes and then integrated into a test apparatus. The measurement is performed with ultrafiltrated and deionized water that is temperature-controlled to 37°C. The test cell is completely immersed in temperature-controlled water during the measurement. The test pressure upstream of the test cell is set at 200±2 mbar. The measurement is a dead-end method. The test cell is first conditioned for 900 seconds under test pressure. The actual measurement time is 60 seconds, in which the permeate produced during the measurement is volumetrically determined.

The $UFR_{water}$ is determined according to the following formula:

$$UFR_{Wasser} = \frac{V_W}{\frac{\Delta t}{3600} \cdot A \cdot \left(\frac{P_O + P_E}{2}\right) \cdot f_{torr}} \quad [ml/(h\ m^2\ mmHg)]$$

In which:

Vw = volume of water [ml] flowed through the membrane specimen during the measurement time
$\Delta t$ = measurement time [s]
A = effective area [$m^2$]
$P_0$ = test pressure [mbar] (pressure upstream of the test cell)
$P_E$ = end pressure [mbar] (pressure downstream of the test cell)
$f_{torr}$ = 1/1.33322; conversion [mbar] to [mmHg].

The invention will be explained in more detail based on the following examples and figures; the area of the invention is, however, not limited by them.

Figure 1 is a representation of a first embodiment of the device according to the invention

Figure 2 is a representation of a second embodiment of the device according to the invention

Figure 3 is a representation of a third embodiment of the device according to the invention

The schematic representation according to Figure 1 shows a preferred embodiment of the device 1 for multistage treatment of a fluid comprising a first fluid treatment compartment 2 and a second fluid treatment compartment 3 being arranged in sequence. The first fluid treatment compartment 2 has a first cylindrical shell 4 with a first shell end 5 and a second shell end 6. Within the cylindrical shell 4 a plurality of first hollow fiber membranes 7 is arranged, wherein the first hollow fibers membranes 7 have a first end 8 and a second end 9 and wherein the first hollow fiber membranes 7 are arranged in first cylindrical shell 4 such that they extend between first shell end 5 and shell second end 6. The first hollow fibers membranes 7 are embedded with their first end 8 in a first tube sheet 10 bonded to an interior side of shell 4 at the first end 5 of first shell 4 and with their second ends 6 in a second tube sheet 11 bonded to the interior side of first shell 4 at the second end 6 of first shell 4. A first outer space 12 is formed around the first hollow fiber membranes 7 which is limited by the interior side of first shell 4 and first and second tube sheets 10, 11. The first hollow fibers membranes 7 pass with their ends 8, 9 through the respective tube sheets 10, 11 and are open at their ends 8, 9.

Attached to the first end 5 of first shell 4 the first fluid treatment compartment 2 has a first end cap 13 with a first fluid port 14 , which first fluid port 14 is in fluid connection with the lumina of the first hollow fiber membranes 7 at their first ends 8 via a first headspace 15 formed between first end cap 13 and first tube sheet 10. The first fluid treatment compartment 2 has at least one opening 16 in the first shell 4 between first and second tube sheets 10, 11 to permit fluid flow into or out of the first outer space 12 surrounding the first hollow fiber membranes 7.

The second fluid treatment compartment 3 comprises a second cylindrical shell 17 having a third end 18 and a fourth end 19. Within the second cylindrical shell 17 a plurality of fibers 20 formed into a bundle is arranged such that the fibers 20 extend between third and fourth end 18, 19 of the second cylindrical shell 17. In the present embodiment, fibers 20 are embedded with their ends in a third tube sheet 21 and a fourth tube sheet 22 bonded to an interior side of the second shell 17 at ends 18, 19 of second shell 17 such that a second outer space 23 is formed around the fibers 20. In the embodiment shown in Fig. 1 the fourth tube sheet 22 forms the end piece fluid-tightly delimiting the second cylindrical shell 17. Second outer space 23 is limited by second shell 17 and tube sheets 21, 22 at the ends 18, 19 of second shell 17.

The second fluid treatment compartment 3 has in tube sheet 21 openings 24 arranged in an outer ring section of tube sheet 21 through which openings 24 fluid flow is possible into or out of second outer space 23. Openings 24 may be through-holes or elongated slits or may be formed by tubes embedded into third tube sheet 21 together with fibers 20, wherein the tubes extend through tube sheet 21 and are open at both their ends.

In the embodiment according to Fig. 1, the second fluid treatment compartment 3 has a fluid port 25 in the second cylindrical shell 17 at fourth end 19, which fluid port 25 is in fluid communication with second outer space 23 and is adapted to permit fluid flow into or out of second outer space 23. The second cylindrical shell 17 may have in addition a further fluid port 26 at third end 18 which may be advantageous in cases a washing fluid is introduced into second outer space 23 which flows through the second outer space 23 or for deaeration of the second outer space 23.

First fluid treatment compartment 2 and second fluid treatment compartment 3 of the embodiment shown in Fig. 1 are fluid-tightly connected to each other by connecting element 27 so as to form an integrated unit. Connecting element 27 e.g. may be a union nut or a cylindrical shell segment to which first and second cylindrical shell 4, 17 are adhesively bonded. The connection of first and second fluid treatment compartment 2, 3 is designed such that a second headspace 28 is formed between second tube sheet 11 and third tube sheet 21 and a fluid path is formed between the lumina of hollow fibers 7 of the first fluid treatment compartment 2 and second outer space 23 of the second fluid treatment compartment 23.

During use, with the device of the invention a cascading treatment, i.e. a treatment in consecutive manner of a fluid flowing through the device 1 can be performed, e.g. with a first fluid treatment step in form of a filtration in first fluid treatment compartment 2 and a second step in form of an adsorptive treatment in second fluid treatment compartment 3 or vice versa, depending on the flow direction through the device. The fluid may first pass through device 1 of the invention in such a way that it first flows through first fluid port 14 in first end cap 13 into first headspace 15 and from there into the lumina of first hollow fiber membranes 7 of the first fluid treatment compartment 2. Part of the fluid flowing through the lumina of first hollow fiber membranes 7 may flow as permeate through the walls of the first hollow fiber membranes 7 into the first outer space 12 of the first fluid treatment compartment 2, by that the fluid to be treated may undergo a filtration step. The permeate may leave the first outer space 12 via a side port 16 in the first cylindrical shell 4 positioned between first tube sheet 10 and second tube sheet 11.

The retentate flows from the lumina of the first hollow fiber membranes 7 into second headspace 28 between second tube sheet 9 and third tube sheet 21. From there, it streams through openings 24 in third tube sheet 21 into the second outer space 23 surrounding fibers 20 in second treatment compartment 3 and flows over the plurality of fibers 20. When flowing over fibers 20 in second fluid treatment compartment 3, the retentate of the fluid to be treated may be treated in a second step e.g. by an adsorption process. The fluid - after having been treated in the second treatment compartment 3 - is finally removed from second treatment compartment 3 via fluid port 25.

Fig. 2 shows in a schematic representation another preferred embodiment of the present device 1 comprising a first fluid treatment compartment 2 and a second fluid treatment compartment 3 being arranged in sequence. The first fluid treatment compartment 2 of the embodiment of Fig. 2 corresponds to the one shown in Fig. 1 and the same components are designated with the same reference numbers and a detailed description of corresponding elements is not repeated.

While the first fluid treatment compartment 2 of the embodiment of Fig. 2 corresponds to the one of Fig. 1, the second fluid treatment compartment 3 is different. As far as the elements of second fluid treatment compartment 3 of the embodiment of Fig. 2 correspond to those of Fig. 1, same components are designated with the same reference numbers.

In the embodiment of Fig. 2, the second fluid treatment compartment 3 has a central perforated core tube 29 for guiding the fluid to be treated from the second head space 28 into the second outer space 23 of the second fluid treatment compartment 3. This central perforated core tube 29 has a hollow core, an open end 30 and a closed end 31 and is embedded into the third tube sheet 21 with its open end 30 being in fluid communication with the second headspace 28. The core of the central perforated core tube 29 is adapted to communicate with the second outer space 23 via perforations 32. The section of the core of the bundle of fibers 20 adjacent to the closed end 31 of the central perforated core tube 29 is closed by a closed core tube segment 33, which may be embedded together with fibers 20 in fourth tube sheet 22.

As in the embodiment shown in Fig. 1, the fibers 20 in Fig. 2 are embedded with their ends facing the fourth end 19 of second cylindrical shell 17 in fourth tube sheet 22 bonded to an interior side of second shell 17. The fourth tube sheet 22 in the embodiment of Fig. 2, however, has openings 34 arranged in an outer ring section of tube sheet 22, through which openings 34 fluid flow is possible into or out of second outer space 23. Openings 34 may be through-holes or elongated slits or may be formed by tubes embedded into fourth tube sheet 22 together with fibers 20, wherein the tubes extend through tube sheet 22 and are open at both their ends. For the embodiment shown in Fig. 2, the second cylindrical shell 17 is closed at the fourth end 19 by a second end cap 35 which forms the end piece.

Between second end cap 35 and fourth tube sheet 22 a third headspace 36 is formed with fluid port 37. Second cylindrical shell 17 may in addition have fluid ports 25, 26 at or nearby ends 18,19 which fluid ports 25, 26 may be advantageous in cases a washing fluid is guided through second outer space 23 or for deaeration of second outer space 23.

In case of the embodiment shown in Fig. 2, a fluid to be treated e.g. after having left the first fluid treatment compartment 2 flows into the second headspace 28 and from there into the core of the central perforated tube 29 via its open end 30. The fluid to be treated leaves the central perforated tube 29 via perforations 32 in the tube wall, enters second outer space 23 and flows across and along fibers 20 arranged in the second fluid treatment compartment 3. The treated fluid flows out of second outer space 23 via openings 34 in tube sheet 22 into third headspace 36 between fourth tube sheet 22 and second end cap 35 and finally leaves the second fluid treatment compartment 3 and thus device 1 via fluid port 37 in end cap 35.

Of course, also flow of a fluid to be treated in the opposite direction is possible, i.e. flow via the fluid port 37 into third headspace 36 and through the openings or tubes in the fourth tube sheet into the second outer space, etc.

Fig. 3 shows a further preferred embodiment of the present device 1. As for the embodiment of Fig. 2, the first fluid treatment compartment corresponds to the one shown in Fig. 1 and same components are designated with same reference numbers and a detailed description regarding these components is not repeated. In the device 1 of Fig. 3, the fibers 20 placed into the second fluid treatment compartment 3 are hollow fibers 38. Hollow fibers 38 are arranged in U-shape with their terminal ends being embedded in third tube sheet 21 and with closed U-shaped ends 39 opposite the

terminal ends and being directed towards the fourth end 19 of second cylindrical shell 17. The closed U-shaped ends 39 terminate in second outer space 23. The terminal ends of the hollow fibers 38 are embedded in the third tube sheet 21 such that the lumina of the hollow fibers 38 are closed by the third tube sheet 21. In the embodiment of Fig. 3, the second cylindrical shell 17 is closed by a fourth tube sheet 22 forming the end piece fluid-tightly delimiting second cylindrical shell 17. A second outer space 23 is limited by the second shell 17 and tube sheets 21, 22.

A fluid, e.g. entering the second outer space 23 via openings 24 in third tube sheet 21 and flowing through second outer space 23 along the hollow fibers 38 preferably exits the second outer space 23 via fluid port 25 in the second cylindrical shell 17 at fourth end 19, which fluid port 25 is in fluid communication with second outer space 23. Second cylindrical shell 17 may have in addition a further fluid port 26 at third end 18 which may be advantageous in cases a washing fluid is guided through second outer space 23 or e.g. for deaeration of the second outer space 23.

## Claims

**1.** Device for multi-stage treatment of a fluid comprising at least a first fluid treatment compartment and a second fluid treatment compartment being arranged in sequence,

- the first fluid treatment compartment comprising

  - a first cylindrical shell having a first end and a second end,
  - a plurality of first hollow fiber membranes based on an organic polymer, each said first hollow fiber membranes having a first end and a second end, a lumen and a wall surrounding the lumen,
  - wherein said first hollow fiber membranes are arranged in the first cylindrical shell and are extending between the first end and the second end,
  - wherein said first hollow fibers membranes are embedded with their first end in a first tube sheet bonded to an interior side of the shell at the first end of the first shell and with their second ends in a second tube sheet bonded to the interior side of the first shell at the second end of the first shell, such that a first outer space is formed around the first hollow fiber membranes which is limited by the interior side of the first shell and the first and second tube sheets,
  - wherein the first hollow fibers membranes pass with their ends through the respective tube sheets and are open at their ends,
  - wherein the first fluid treatment compartment has attached to the first end of the first shell a first end cap with a first fluid port, which first fluid port being in fluid connection with the lumina of the first hollow fiber membranes at their first ends via a first headspace formed between first end cap and first tube sheet,
  - wherein the first fluid treatment compartment has at least one opening in the first shell between first and second tube sheets to permit fluid flow into or out of the first outer space surrounding the first hollow fiber membranes,

- the second fluid treatment compartment comprising

  - a second cylindrical shell having a third end and a forth end, wherein the fourth end of the second cylindrical shell being delimited by an end piece,
  - a plurality of fibers based on an organic polymer which fibers having a length extension and ends,
  - wherein said fibers are arranged in the second cylindrical shell such that they extend between third and fourth end of the second cylindrical shell and
  - wherein the fibers are at least embedded with one of their ends in a tube sheet bonded to an interior side of the second shell at one of the ends of the second shell such that a second outer space is formed around the fibers which is limited at least by the second shell and the end piece at the fourth end of the second shell,
  - wherein the second fluid treatment compartment has at least one fluid port, which at least one fluid port of the second fluid treatment compartment being in fluid communication with the second outer space and being adapted to permit fluid flow into or out of the second outer space surrounding the fibers arranged in the second shell, and

wherein the device is characterized in that_first fluid treatment compartment and second fluid treatment compartment are connected so as to form an integrated unit, wherein

  - second end of first cylindrical shell and third end of second cylindrical shell are fluid-tightly connected to each other such that at the transition between the first fluid treatment compartment and the second fluid

treatment compartment a fluid path is formed between the lumina of the hollow fibers of the first fluid treatment compartment and the second outer space of the second fluid treatment compartment.

2. Device according to claim 1, wherein the fibers arranged in the second cylindrical shell are solid fibers.

3. Device according to claim 1, wherein the fibers in the second cylindrical shell are arranged in U-shape, thus having terminal ends and a closed U-shaped fiber end opposite the terminal ends.

4. Device according to claim 1,

- wherein the fibers of the second fluid treatment compartment are embedded with their ends facing the third end of the second shell in a third tube sheet bonded to an interior side of the second shell at the third end of the second shell such that the second outer space formed around the fibers is limited by the second shell, the third tube sheet and the end piece at the fourth end of the second shell, with the third tube sheet having an interior face facing the second outer space and an exterior face facing outward, and
- wherein second end of first cylindrical shell and third end of second cylindrical shell are fluid-tightly connected to each other such that a second headspace is formed between second tube sheet and third tube sheet,
- wherein the third tube sheet has at least one opening therethrough forming a fluid connection between the second outer space and the second headspace so that the lumina of the first hollow fiber membranes of the first fluid treatment compartment, the second head space and the second outer space are in fluid communication.

5. Device according to claim 4, wherein the fibers arranged in the second cylindrical shell are embedded with their ends facing the fourth end of the second cylindrical shell in a fourth tube sheet which forms the end piece.

6. Device according to claim 5, wherein the fourth tube sheet closes the fourth end of the second cylindrical shell fluid-tightly and wherein the at least one fluid port of the second fluid treatment compartment is a side port attached to the second cylindrical shell between third tube sheet and fourth tube sheet.

7. Device according to claim 5, wherein the fourth tube sheet comprises at least one opening therethrough, wherein a second end cap is attached to the fourth end of the second cylindrical shell such that a third headspace is formed between fourth tube sheet and second end cap, wherein second outer space and third head-space are in fluid communication via the at least one opening in the fourth tube sheet and wherein the at least one fluid port of the second fluid treatment compartment is attached to the second end cap.

8. Device according to claim 4, wherein the fiber ends facing the fourth end of the second cylindrical shell terminate within the second outer space and the second cylindrical shell has attached to its fourth end a second end cap forming the end piece at the fourth end.

9. Device according to claim 8, wherein the at least one fluid port of the second fluid treatment compartment is attached to the second end cap.

10. Device according to claim 1,

- wherein the fibers of the second fluid treatment compartment are embedded with their ends facing the fourth end of the second shell in a fourth tube sheet bonded to an interior side of the second shell at the fourth end of the second cylindrical shell which fourth tube sheet forming the end piece at the fourth end of the second cylindrical shell,
- wherein the ends of the fibers facing the third end of the second cylindrical shell terminate within the second outer space and are free of an embedding,
- wherein second end of first cylindrical shell and third end of second cylindrical shell are fluid-tightly connected to each other such that the second outer space formed around the fibers is limited by the second shell, the fourth tube sheet and the second tube sheet of the first shell and
- wherein the lumina of the first hollow fiber membranes of the first treatment compartment are in direct fluid communication with the second outer space.

11. Device according to claim 10, wherein the fourth tube sheet closes the fourth end of the second cylindrical shell fluid-tightly and wherein the at least one fluid port of the second fluid treatment compartment is a side port attached to the second cylindrical shell between third end of the second shell and fourth tube sheet.

12. Device according to claim 10, wherein the fourth tube sheet comprises at least one opening therethrough, wherein a second end cap is attached to the fourth end of the second cylindrical shell such that a third headspace is formed between fourth tube sheet an second end cap and second outer space and third head-space are in fluid communication via the at least one opening in the fourth tube sheet and wherein the at least one fluid port of the second fluid treatment compartment is attached to the second end cap.

13. Device according to claim 1, wherein the hollow fiber membranes of the first fluid treatment compartment are adapted to remove cytokines from a blood sample flowing through the lumina of the hollow fiber membranes.

14. Device according to claim 1, wherein the hollow fiber membranes of the first fluid treatment compartment have a separation characteristic such that their sieving coefficient for albumin is in the range from 0,015 to 0,35.

15. Device according to claim 1, wherein the fibers of the second fluid treatment compartment are adapted to remove granulocytes and monocytes by adsorption from a blood sample flowing through the second outer space.

16. Process for a multi-staged treatment of a fluid, wherein a device according to one or more of claims 1 to 15 is used and the fluid is guided in a consecutive manner through the first and the second fluid treatment compartment of the device or vice versa.

17. Process according to claim 16, wherein the fluid is treated in the first fluid treatment compartment in a filtration step and in the second fluid treatment compartment in an adsorption step.

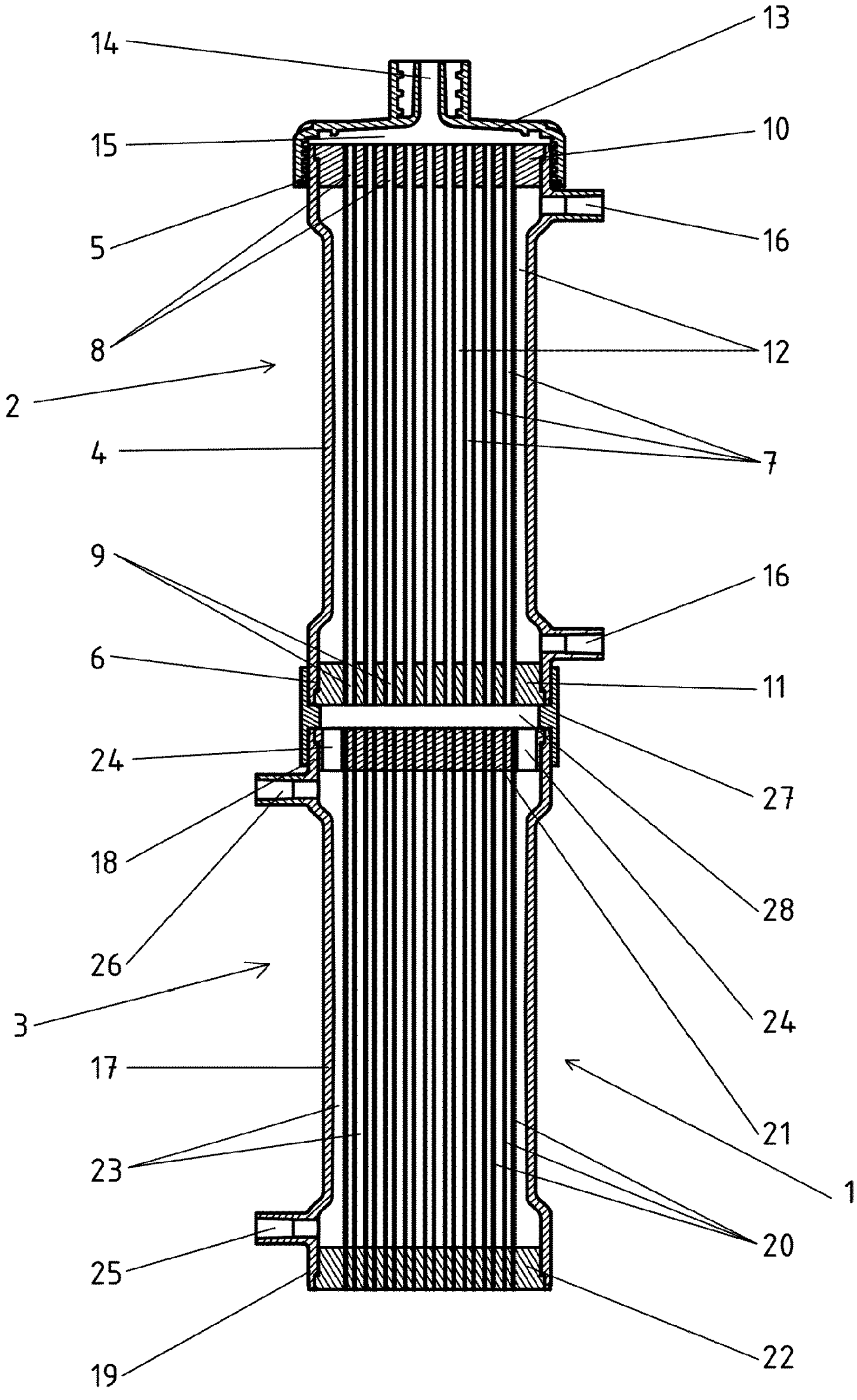
14
13
15
10
5
16
8
12
2
4
7
9
16
6
11
24
27
18
28
26
3
24
17
21
23
1
25
20
19
22

Fig. 1

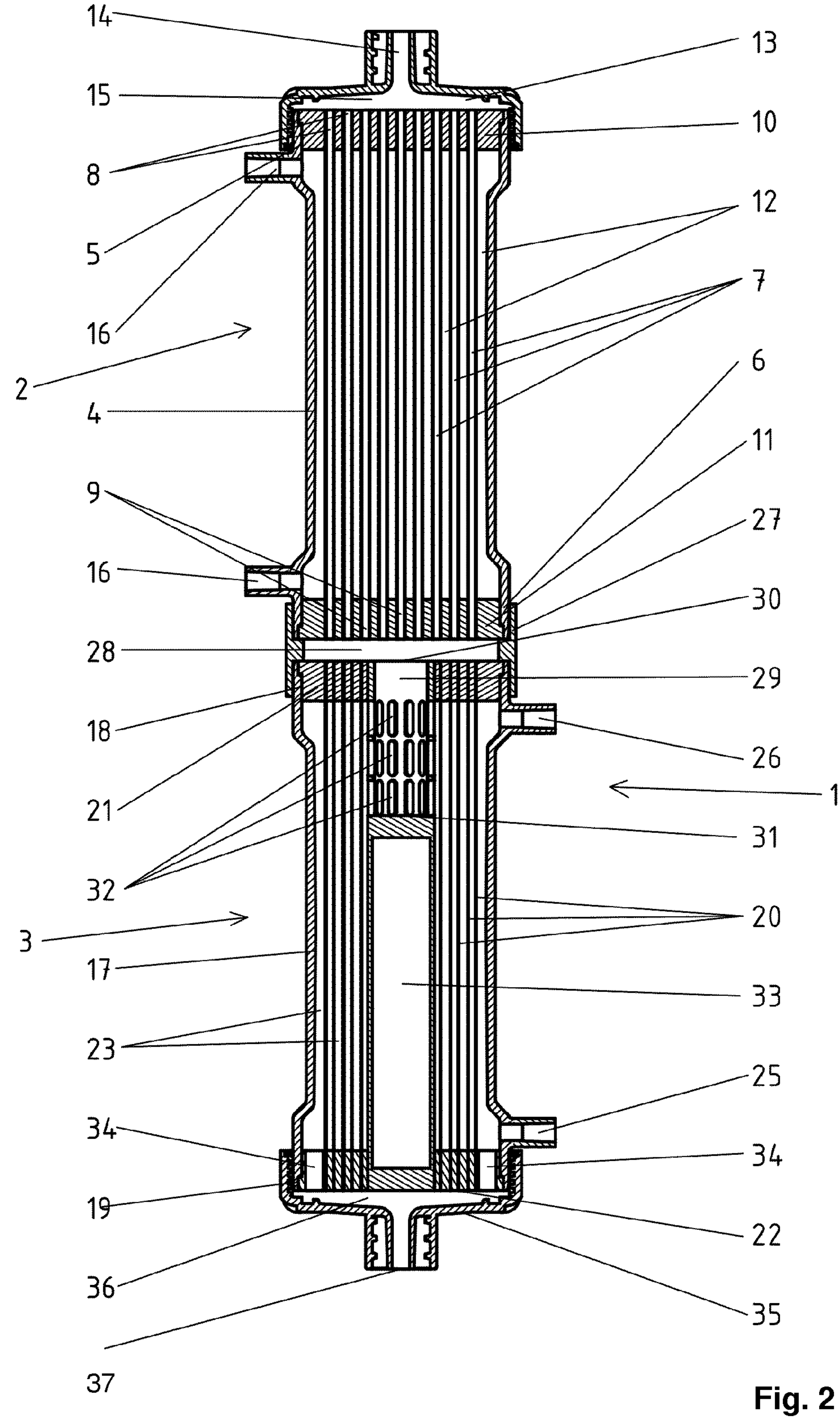
14
13
15
10
8
12
5
7
16
2
6
4
11
9
27
16
30
28
29
18
26
1
21
31
32
20
3
17
33
23
25
34
34
19
22
36
35
37

Fig. 2

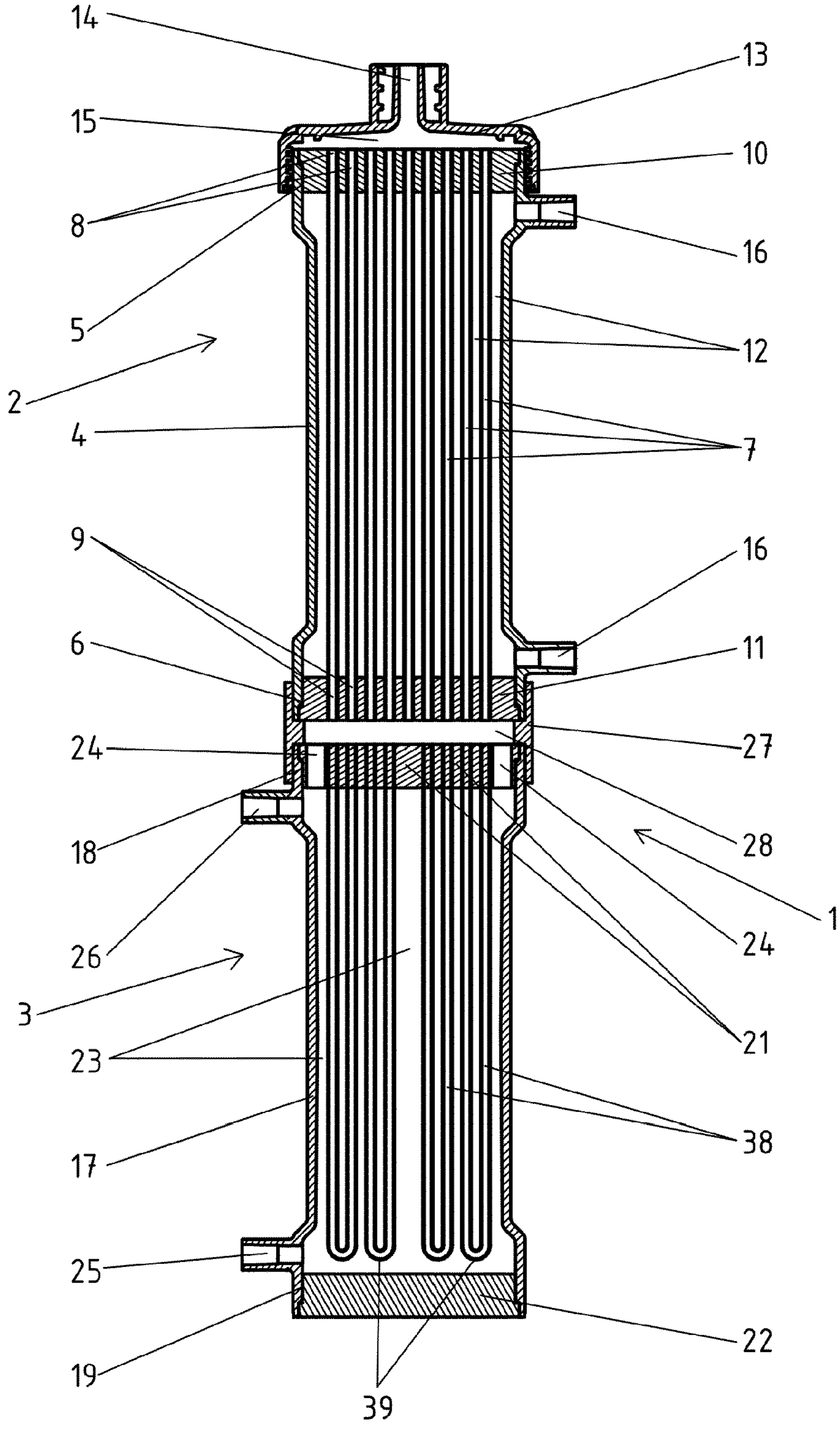
14
13
15
10
8
16
5
12
2
4
7
9
16
6
11
24
27
18
28
1
26
24
3
21
23
38
17
25
22
19
39

Fig. 3

Europäisches Patentamt
European Patent Office
Office européen des brevets

# EUROPEAN SEARCH REPORT

Application Number
EP 17 19 7384

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2016/030357 A1 (3M INNOVATIVE PROPERTIES CO [US]) 3 March 2016 (2016-03-03) * claim 1 * * claim 9 * * claim 10 * * claim 12 * * claim 7 * * page 16, line 24 * ----- | 1-17 | INV. B01D61/28 A61M1/34 A61M1/36 B01D63/02 B01D69/02 |
| X<br>Y | US 2015/283315 A1 (CHO TAEBEOM [KR]) 8 October 2015 (2015-10-08) * claim 1 * * figure 1 * * figure 3 * * paragraph [0034] * ----- | 1,3-17<br>2 | |
| X<br>Y | US 2017/095603 A1 (CHO TAEBEOM [KR]) 6 April 2017 (2017-04-06) * claim 1 * * figure 2B * * figure 3B * ----- | 1,3-17<br>2 | |
| Y | WO 2011/068897 A1 (EXTHERA MEDICAL LLC [US]; WARD ROBERT S [US]; MCCREA KEITH R [US]; LAR) 9 June 2011 (2011-06-09) * page 12, line 4 - line 12 * ----- | 2 | TECHNICAL FIELDS SEARCHED (IPC)<br>B01D<br>A61M |

The present search report has been drawn up for all claims

1

| Place of search | Date of completion of the search | Examiner |
|---|---|---|
| Munich | 9 March 2018 | Hoyer, Michael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO.

EP 17 19 7384

This annex lists the patent family members relating to the patent documents cited in the above-mentioned European search report. The members are as contained in the European Patent Office EDP file on
The European Patent Office is in no way liable for these particulars which are merely given for the purpose of information.

09-03-2018

| Patent document cited in search report | Publication date | Patent family member(s) | Publication date |
|---|---|---|---|
| WO 2016030357 A1 | 03-03-2016 | CN 106659834 A | 10-05-2017 |
| | | EP 3185926 A1 | 05-07-2017 |
| | | JP 2017525489 A | 07-09-2017 |
| | | US 2017266362 A1 | 21-09-2017 |
| | | WO 2016030357 A1 | 03-03-2016 |
| US 2015283315 A1 | 08-10-2015 | KR 20150114887 A | 13-10-2015 |
| | | US 2015283315 A1 | 08-10-2015 |
| US 2017095603 A1 | 06-04-2017 | KR 20150114886 A | 13-10-2015 |
| | | US 2017095603 A1 | 06-04-2017 |
| WO 2011068897 A1 | 09-06-2011 | AU 2010326028 A1 | 21-06-2012 |
| | | CA 2782311 A1 | 09-06-2011 |
| | | CA 2967094 A1 | 09-06-2011 |
| | | CN 102740859 A | 17-10-2012 |
| | | CN 106178161 A | 07-12-2016 |
| | | EP 2509604 A1 | 17-10-2012 |
| | | HK 1177151 A1 | 14-07-2017 |
| | | JP 5925693 B2 | 25-05-2016 |
| | | JP 6072189 B2 | 01-02-2017 |
| | | JP 2013512078 A | 11-04-2013 |
| | | JP 2016034966 A | 17-03-2016 |
| | | KR 20120103671 A | 19-09-2012 |
| | | KR 20150013310 A | 04-02-2015 |
| | | US 2011184377 A1 | 28-07-2011 |
| | | US 2014231357 A1 | 21-08-2014 |
| | | US 2016331886 A1 | 17-11-2016 |
| | | WO 2011068897 A1 | 09-06-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016030357 A
- WO 201603057 A
- US 20090060890 A
- US 20120322138 A
- EP 0285812 A